# EUROPEAN PATENT SPECIFICATION

(11) **EP 1 669 359 B1**
(45) Date of publication and mention of the grant of the patent: **29.08.2007**
(21) Application number: 06100356.2
(22) Date of filing: 10.06.2003
(51) Int. Cl.: C07D 495/04

(54) **A process for the preparation of olanzapine and an intermediate therefor**
Verfahren zur Herstellung von Olanzapine und ein Zwischenprodukt dafür
Procédé de préparation d'olanzapine et intermédiaire utilise

(30) Priority: 20.06.2002 PL 35464202
(43) Date of publication of application: 14.06.2006
(62) Divisional of application: 03732782.2
(73) Proprietor: Adamed SP. Z O.O., 05-152 Czosnwó k/Warszawy (PL)
(72) Inventor: Majka, Zbigniew, 39-102, Lubzina (PL); Stawinski, Tomasz, 05-462, Wiazowna (PL); Rechnio, Justyna, 050530, Góra Kalwaria (PL); Wieczorek, Maciej, 05-092, Lomianki (PL)
(74) Representative: HOFFMANN EITLE

(56) References cited:
- EP-A- 0 454 436
- GB-A- 1 533 235
- CALLIGARO D O ET AL: "The synthesis and biological activity of some known and putative metabolites of the atypical antipsychotic agent olanzapine (LY170053)" BIOORGANIC & MEDICINAL CHEMISTRY LETTERS, OXFORD, GB, vol. 7, no. 1, 7 January 1997 (1997-01-07), pages 25-30, XP004135960 ISSN: 0960-894X
- DATABASE BEILSTEIN [Online] 1987, XP002375645 Database accession no. 5557715 & VEJDELEK, ZDENEK; PROTIVA, MIROSLAV: COLLECT. CZECH. CHEM. COMMUN, vol. 52, no. 7, 1987, pages 1834-1840,

## Description

### Technical field

The invention relates to a process for olanzapine preparation, in particular to the process for olanzapine preparation using new *N*-demethylolanzapine derivative as a starting material, to a new *N*-demethylolanzapine derivative being an intermediate for olanzapine preparation and to a process for the preparation of this new *N*-demethylolanzapine derivative using *N*-demethylolanzapine as a starting material.

### Background of the invention

Olanzapine, or 2-methyl-4-[4-methyl-1-piperazinyl]-10H-thieno[2,3-*b*][1,5]-benzodiazepine, is a recognised drug acting on the central nervous system and is known, among others, from EP 0454436. In GB 1533235 there are disclosed processes for the preparation of alkyl-piperazine benzodiazepine derivatives involving the alkylation of the piperazine ring.

In EP 0454436 there are disclosed processes for olanzapine preparation. One of the known procedures consists in reduction and cyclization reaction of 2-(2-nitroanilino)-5-methylthiophen-3-carbonitrile with stannous chloride SnCl₂ in an aqueous-alcoholic solution of hydrogen chloride, followed by a reaction of thus formed 4-amino-2-methyl-10H-thieno[2,3-b][1,5]-benzodiazepine with *N-*methylpiperazine in an organic solvent such as anisole, toluene, dimethylformamide or dimethylsulphoxide (DMSO), preferably at a temperature from 100 to 150°C, to produce olanzapine. Another of the known procedures consists in cyclization of 1-{[2-(2-aminoaniline)-5-methylthiophen-3-yl]carbonyl}-4-methylpiperazine, which is in turn obtained from methyl cyanoacetate in a series of laborious steps requiring specific complex reaction conditions, reactants and reducing agents as well as high-boiling and hard to remove solvents, like toluene, DMF, DMSO, etc.. The reaction yields of the prior-art processes are not high. Further disadvantage of the prior-art processes is generation of impurities which have to be removed by repeated crystallizations, what has adverse effect on the process efficiency.

The aim of this invention was to develop a new process for olanzapine preparation, which would not require the use of hard-to-remove organic solvents.

Further aim was to develop a new process for olanzapine preparation, which would involve more simple chemical procedures, while allowing to obtain high yields of the final product having satisfactory purity.

To remove the aforementioned deficiencies of the prior art the applicants have developed new processes for olanzapine preparation, wherein new *N*-demethylolanzapine derivative, that is 2-methyl-4-(4-formylpiperazin-1-yl)-10H-thieno[2,3-*b*][1,5]benzodiazepine, obtainable from 2-methyl-4-piperazin-1-yl-10H-thieno-[2,3-*b*][1,5]benzodiazepine is used as the starting material.

According to the processes of the invention crude olanzapine is produced which is as pure as the one obtained by prior art processes, in mild conditions with relatively short reaction times and low reaction temperatures. The use of low-volatile solvents and generation of great amounts of impurities is avoided.

*N*-Demethylolanzapine is a compound known as an olanzapine metabolite and is described by Calligaro et al., Biorg. & Med. Chem. Letters, 1, 25-39, (1997), and in the publication of the international patent application WO00/30650. This compound can readily be made by a reaction of the known compound 4-amino-2-methyl-10H-thieno[2,3-*b*][1,5]benzodiazepine with piperazine as described by Calligaro et al., Biorg. & Med. Chem. Letters, 1, 25-39, (1997).

### Detailed description of the invention

In a first aspect, the invention provides a process for the preparation of olanzapine (2-methyl-4-[4-methyl-1-piperazinyl]-10H-thieno-[2,3-*b*][1,5]-benzodiazepine), of the structural formula I, 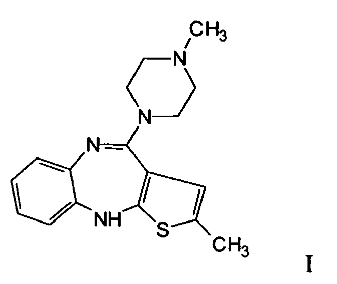 which process comprises reduction of 2-methyl-4-(4-formyl-1-piperazinyl)-10H-thieno[2,3-*b*][1,5]-benzodiazepine (*N*-demethyl-*N-*formylolanzapine) of formula III

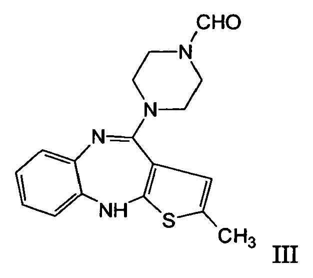

Preferably, the said reduction reaction is carried out with a Group I or II metal borohydride as said reducing agent in an aqueous medium at a temperature in the range from -10°C to +20°C.

Preferably, an alkali-metal borohydride, in particular sodium borohydride, is used.

The preferred reaction temperature is a temperature in the range from 0°C to 5°C.

The reduction with borohydride is conducted using the procedure which as such is known in the art. Typically, the reaction is carried out in an aqueous medium, in the presence of acetic acid and sodium acetate as a buffer. The borohydride is added portionwise to the cooled reaction mixture containing N-demethyl-N-formylolanzapine. After reaction is complete, the reaction mixture is made alkaline, thereby crude olanzapine precipitates and the precipitate is subsequently isolated.

Preferably the *N*-demethyl-*N*-formylolanzapine is produced in a reaction of *N*-demethylolanzapine with ethyl formate, optionally in the presence of a solvent.

*N*-Demethyl-*N*-formylolanzapine is a new compound, not disclosed in the prior-art.

Accordingly, in its second aspect the invention provides also a new compound *N*-demethylolanzapine derivative, that is 2-methyl-4-(4-formyl-1-piperazinyl)- 10H-thieno[2,3-*b*][1,5]-benzodiazepine (*N*-demethyl-*N*-formylolanzapine) of formula III:

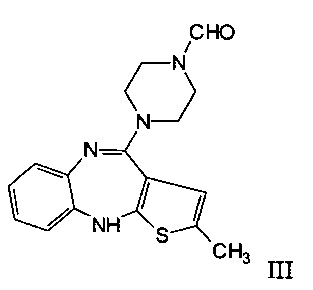

As stated above, *N*-demethyl-*N*-formylolanzapine is useful as an intermediate in the olanzapine preparation. Olanzapine is prepared by reduction of *N*-demethyl-*N*-formylolanzapine.

In the third aspect the invention provides a process for the preparation of 2-methyl-4-(4-formyl-1-piperazinyl)-10H-thieno[2,3-*b*][1,5]-benzodiazepine (*N*-demethyl-*N*-formylolanzapine) of formula III, 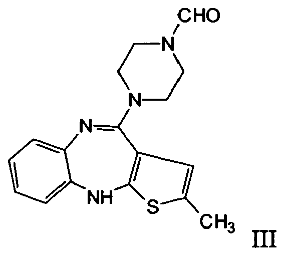 which process comprises reaction of 2-methyl-4-piperazin-1-yl-10H-thieno-[2,3-*b*][1,5]-benzodiazepine (*N*-demethylolanzapine) of formula II 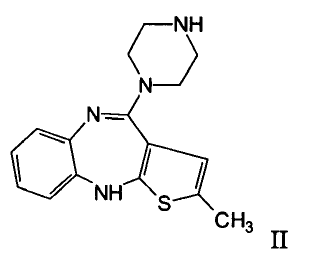 with ethyl formate.

The manner of carrying out the reaction with ethyl formate is simple and known in the art as such. Typically ethyl formate, which also serves as a reaction solvent, is used in considerable excess (about 70 moles of ethyl formate per 1 mole of N-demethylolanzapine). The amount of the ethyl formate used can be decreased by half, if an inert organic solvent, like tetrahydrofuran, is introduced as a co-solvent. The reaction is carried out at the reflux temperature of the reaction medium. On cooling *N*-demethyl-*N-*formylolanzapine precipitates from the reaction mixture.

The invention will be described in more detail with reference to the following, non-limiting examples, which should not be construed as the limitation of its scope.

### Example 1

### Preparation of N-demethyl-N-formylolanzapine (i.e. 2-methyl-4-(4-formyl-1-piperazinyl)-10H-thieno[2,3-b][1,5]-benzodiazepine)

A solution of 5 g (16.8 mmol) of *N*-demethylolanzapine in 100 ml of ethyl formate and 100 ml of tetrahydrofuran was kept under reflux for 20 h. Subsequently, the mixture was cooled in an ice-bath. The pale beige precipitate formed was filtered off and dried in the air. The product, 2-methyl-4-(4-formyl-1-piperazinyl)-10H-thieno[2,3-*b*][1,5]-benzodiazepine was obtained in a 4 g quantity (yield: 72,9%). The structure of the compound produced was confirmed by the NMR and MS analyses.
NMR (CDCl₃) δ ppm: 2.32 (d, J = 1.2 Hz, 3H); 3.38-3.69 (m, 8H); 4.88 (s, 1H); 6.29 (d, J = 1.2 Hz, 1H); 6.57-7.07 (m, 4H); 8.11 (s, 1H), MS: 255 (100%), M-1 = 325 (58%), M = 326 (12%).

### Example 2 Preparation of olanzapine by reduction of N-demethyl-N-formylolanzapine

A mixture of 2-methyl-4-(4-formyl-1-piperazinyl)-10H-thieno-[2,3-*b*][1,5]-benzodiazepine (3 g, 9.2 mmol), sodium acetate (1 g, 12.2 mmol), 15 ml of glacial acetic acid and 15 ml of distilled water was cooled in an ice-bath to 0°C. Under vigorous stirring sodium borohydride (4 g, 9.4 mmol) was added portionwise. The stirring and cooling were continued for about 0.5 h after completion of the borohydride addition. Subsequently, the mixture was made alkaline to a pH = ca. 9 with 2N NaOH aq. The light-yellow precipitate formed was filtered off, washed with water and dried in an air-circulation oven at a temperature of ca. 25°C. Olanzapine was obtained (2.5 g) of an 88% purity as determined by HPLC (yield: 86,9%).

## Claims

1. A process for the preparation of olanzapine of formula I 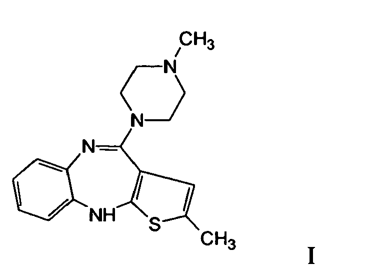 which process comprises reacting 2-methyl-4-piperazin-1-yl-10H-thieno[2,3-*b*][1,5]benzodiazepine (*N*-demethylolanzapine) of formula II 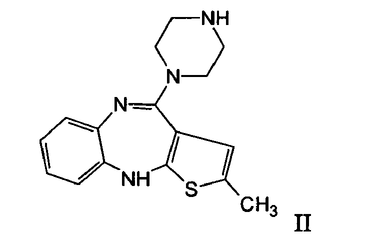 with ethyl formate, optionally in the presence of a solvent, to form 2-methyl-4-(4-formyl-1-piperazinyl)-10H-thieno[2,3-*b*][1,5]benzodiazepine (*N*-demethyl-*N*-formylolanzapine) of formula III 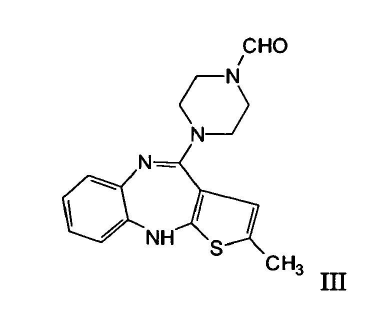 and then reducing *N*-demethyl-*N*-formylolanzapine of formula III thus formed.

2. The process according to Claim 1, wherein said reduction is carried out with a Group I or II metal borohydride in an aqueous medium at a temperature in the range from -10 to +20°C.

3. The process according to Claim 2, wherein said borohydride is an alkali-metal borohydride, in particular sodium borohydride.

4. A process for the preparation of olanzapine of formula I 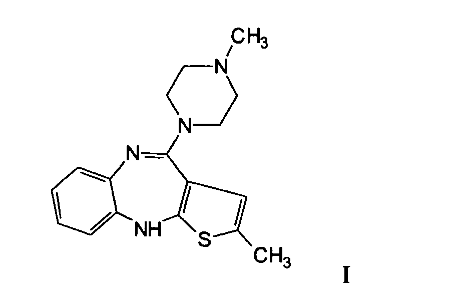 which process comprises reduction of 2-methyl-4-(4-formyl-1-piperazinyl)-10H-thieno[2,3-*b*][1,5]benzodiazepine (*N*-demethyl-*N-*formylolanzapine) of formula III

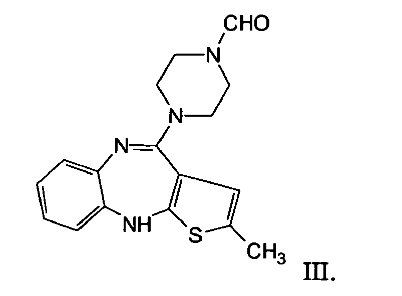

5. The process according to Claim 4, wherein said reduction is carried out with a Group I or II metal borohydride in an aqueous medium at a temperature in the range of-10 to +20°C.

6. The process according to Claim 5, wherein said borohydride is an alkali-metal borohydride, in particular sodium borohydride.

7. The process according to any of the claims 4 to 6, wherein said *N*-demethyl-*N*-formylolanzapine is produced in a reaction of *N*-demethylolanzapine with ethyl formate, optionally in the presence of a solvent.

8. 2-Methyl-4-(4-formyl-1-piperazinyl)-10H-thieno[2,3-*b*]-[1,5]benzodiazepine (*N*-demethyl-*N*-formylolanzapine) of formula III

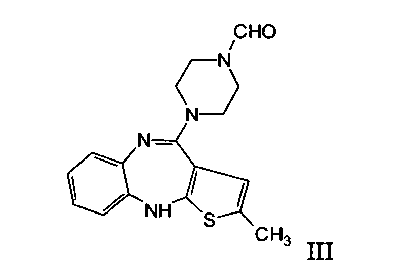

9. A process for the preparation of 2-methyl-4-(4-formyl-1-piperazinyl)-10H-thieno[2,3-*b*][1,5]-benzodiazepine (*N*-demethyl-*N*-formylolanzapine) of formula III, 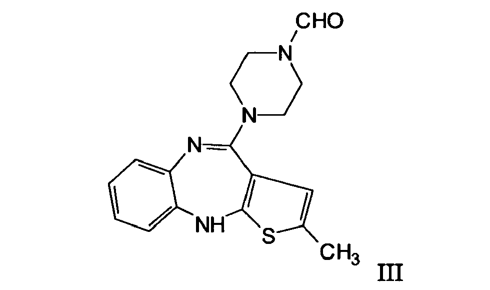 which process comprises reaction of 2-methyl-4-piperazin-1-yl-10H-thieno[2,3-*b*][1,5]benzodiazepine (*N*-demethylolanzapine) of formula II 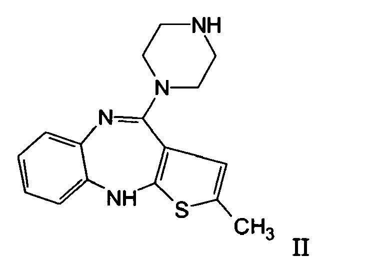 with ethyl formate, optionally in the presence of a solvent.

## Patentansprüche

1. Verfahren zur Herstellung von Olanzapin der Formel I 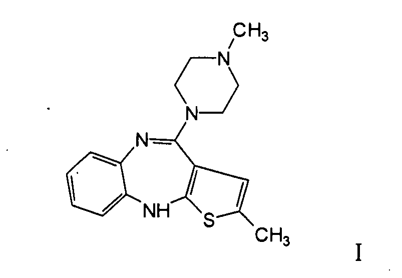 umfassend die Reaktion von 2-Methyl-4-piperazin-1-yl-10H-thieno-[2,3-*b*][1,5]benzodiazepin (*N*-Demethylolanzapin) der Formel II 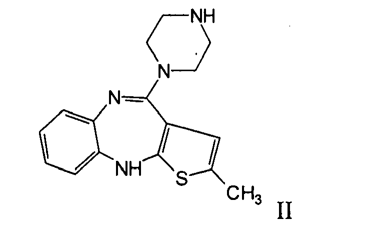 mit Ethylformiat, gegebenenfalls in Gegenwart eines Lösungsmittels, unter Bildung von 2-Methyl-4-(4-Formyl-1-piperazinyl)-10H-thieno[2,3-*b*][1,5]benzodiazepin (*N*-Demethyl -*N*-formylolanzapin) der Formel III 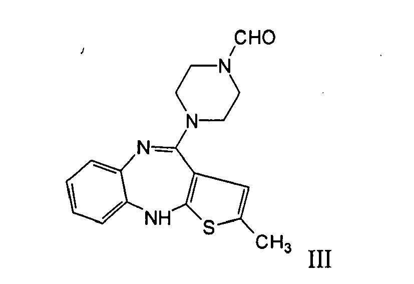 und dann die Reduktion von auf diese Weise erhaltenem *N*-Demethyl-*N*-formylolanzapin der Formel III.

2. Verfahren nach Anspruch 1, wobei die Reduktion mit Borhydrid eines Metalles aus der I oder II Gruppe als Reduktionsmittel, in einem wässrigen Medium bei einer Temperatur im Bereich von -10°C bis +20°C durchgeführt wird.

3. Verfahren nach Anspruch 2, wobei das Borhydrid ein Alkalimetallborhydrid, insbesondere Natriumborhydrid, ist.

4. Verfahren zur Herstellung von Olanzapin der Formel I 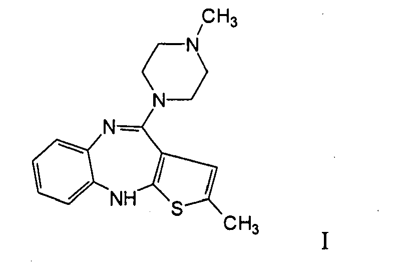 wobei das Verfahren die Reduktion von 2-Methyl-4-(4-formyl-1-piperazinyl)-10H-thieno-[2,3-*b*][1,5]benzodiazepin (*N*-Demethyl-*N*-formylolanzapin) der Formel III 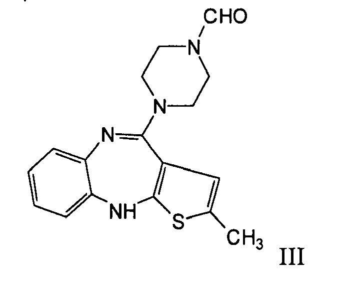 umfasst.

5. Verfahren nach Anspruch 4, wobei die Reduktion mit Borhydrid eines Metalles aus der I. oder II. Gruppe als Reduktionsmittel, im wässrigen Medium bei einer Temperatur im Bereich von -10°C bis +20°C durchgeführt wird.

6. Verfahren nach Anspruch 5, wobei das Borhydrid ein Alkalimetallborhydrid, insbesondere Natriumborhydrid, ist.

7. Verfahren nach einem der Ansprüche 4 bis 6, wobei N-Demethyl-N-formylolanzapin in der Reaktion von *N*-Demethylolanzapin mit Ethylformiat, gegebenenfalls in Gegenwart eines Lösungsmittels, hergestellt wird.

8. 2-Methyl-4-(4-formyl-1-piperazinyl)-10H-thieno[2,3-*b*]-[1,5]benzodiazepin (*N*-demetyl-*N*-formylolanzapin) der Formel III

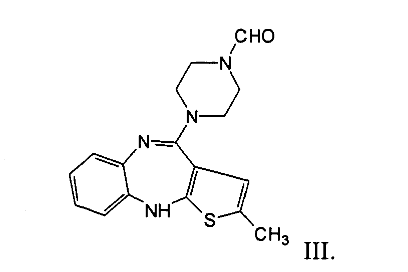

9. Verfahren zur Herstellung von 2-Methyl-4-(4-formyl-1-piperazinyl)-10H-thieno[2,3-*b*][1,5]-benzodiazepin (*N*-Demethyl-*N*-formylolanzapin) der Formel III, 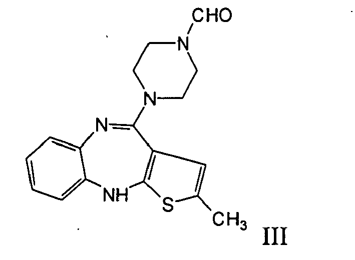 wobei das Verfahren die Reaktion von 2-Methyl-4-piperazin-1-yl-10H-thieno--[2,3-*b*][1,5]benzodiazepin (*N*-Demethylolanzapin) der Formel II 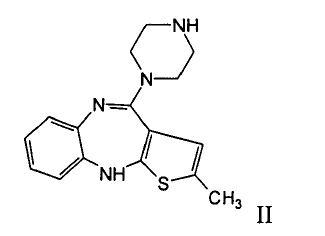 mit Ethylformiat, gegebenenfalls in Gegenwart eines Lösungsmittels, umfasst.

## Revendications

1. Procédé pour la préparation de l'olanzapine de formule I 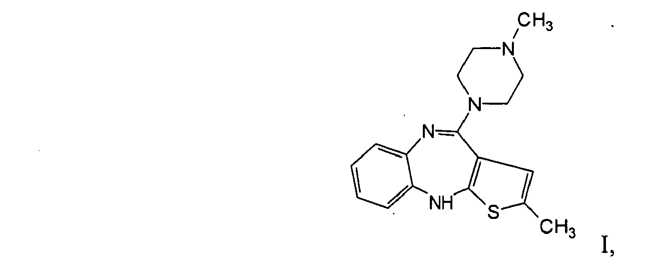 ledit procédé comprenant la mise en réaction de 2-méthyl-4-pipérazin-1-yl-10H-thiéno[2,3-b][1,5]benzodiazépine (N-desméthylolanzapine) de formule II 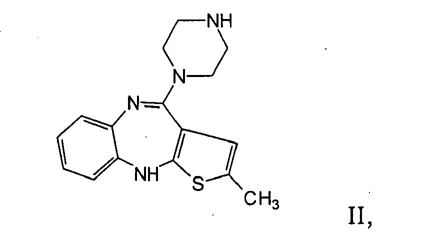 avec du formiate d'éthyle, éventuellement en présence d'un solvant, pour former de 2-méthyl-4-(4-formyle-1-pipérazinyl)-10H-thiéno[2,3-b][1,5]benzodiazépine (N-desméthyl-N-formylolanzapine) de formule III 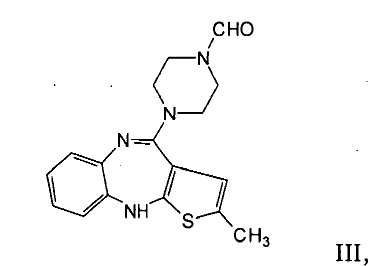 et ensuite réduire N-desméthyl-N-formylolanzapine de formule III ainsi formée.

2. Procédé selon la revendication 1, dans lequel ladite réduction est effectuée avec un borohydrure de métal du Groupe I ou II, dans un milieu aqueux à une température dans la plage de -10° à +20°C.

3. Procédé selon la revendication 2, dans lequel le borohydrure est un borohydrure de métal alcalin, en particulier le borohydrure de sodium.

4. Procédé pour la préparation de l'olanzapine de formule 1 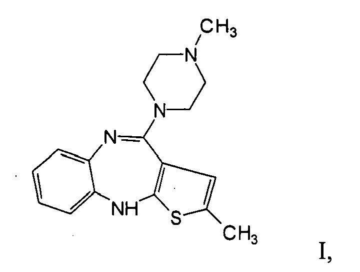 ledit procédé comprenant la réduction de 2-méthyl-4-(4-formyle-1-pipérazinyl)-10H--thiéno[2,3-b][1,5]benzodiazépine (N-desméthyl-N-formylolanzapine) de formule III

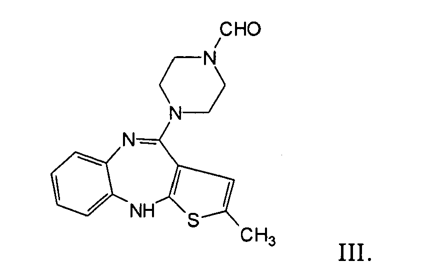

5. Procédé selon la revendication 4, dans lequel ladite réduction est effectuée avec un borohydrure de métal du Groupe I ou II, dans un milieu aqueux à une température dans la plage de -10°C à +20°C.

6. Procédé selon la revendication 5, dans lequel le borohydrure est un borohydrure de métal alcalin, en particulier le borohydrure de sodium.

7. Procédé selon l'une quelconque des revendications 4 à 6, dans lequel la N-desméthyl-N-formylolanzapine est produite par réaction de N-desméthylolanzapine avec du formiate d'éthyle, éventuellement en présence d'un solvant.

8. 2-Méthyl-4-(4-formyle-1-pipérazinyl)-10H-thiéno[2,3-b][1,5]benzodiazépine (N-desméthyl-N-formylolanzapine) de formule III

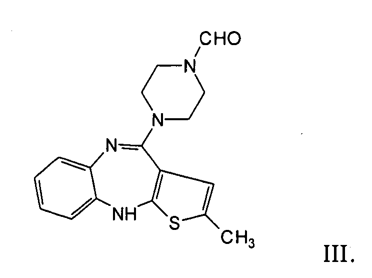

9. Procédé pour la préparation de 2-méthyl-4-(4-formyle-1-pipérazinyl)-10H-thiéno--[2,3-b][1,5]benzodiazépine (N-desméthyl-N-formylolanzapine) de formule III 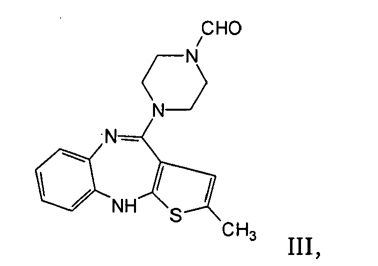 ledit procédé comprenant la réaction de 2-méthyl-4-pipérazin-1-yl-10H-thiéno--[2,3-b][1,5]benzodiazépine (N-desméthylolanzapine) de formule II 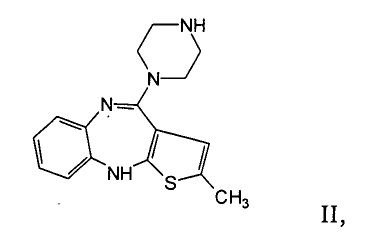 avec du formiate d'éthyle, éventuellement en présence d'un solvant.
